# EUROPEAN PATENT APPLICATION

(11) **EP 3 168 649 A1**
(43) Date of publication of application: **17.05.2017**
(21) Application number: 15860973.5
(22) Date of filing: 29.10.2015
(51) Int. Cl.: G01T 1/161, A61B 6/03, G06T 1/00

(54) **METHOD FOR EVALUATING NUCLEAR MEDICINE IMAGE OF HEAD**

(30) Priority: 21.11.2014 JP 2014236250
(71) Applicant: Nihon Medi-Physics Co., Ltd., Tokyo 136-0075 (JP)
(72) Inventor: MURATA, Akihiro, Tokyo 136-0075 (JP); MORISHITA, Shigenori, Tokyo 136-0075 (JP); DOI, Yoshihiro, Sodegaura-shi Chiba 299-0266 (JP); KOBAYASHI, Ryohei, Sodegaura-shi Chiba 299-0266 (JP)
(74) Representative: V.O.
(86) International application number: PCT/JP2015/080507
(87) International publication number: WO 2016/080168

(57) **Abstract**

To provide a technique for evaluating a nuclear medicine brain image, the technique being hardly affected by the difference in the setting of reference regions. An embodiment of the present invention generally includes: setting a reference region on a region corresponding to the scalp in the nuclear medicine brain image; calculating information on a pixel value in the set reference region; and normalizing, using the information, a pixel value of each pixel included in the nuclear medicine brain image or a value obtained from the pixel value, and outputting the normalized value.

## Description

### Field

The present invention relates to a technique for evaluating a nuclear medicine brain image.

### Background

When a nuclear medicine brain image is used for diagnosing a brain disease, pixel values are normalized by determining a ratio between a site where the distribution of a radiopharmaceutical agent is affected by the disease and a site where the distribution of the radiopharmaceutical agent is regarded as not being affected by the disease. For example, in amyloid imaging for diagnosing Alzheimer's disease, when used for evaluating images of the cortices of the frontal lobe, the temporal lobe, and the parietal lobe, and the posterior cingulated gyrus and the precuneus where amyloid accumulates due to Alzheimer's disease, the following procedure has been performed: a reference region is set on the cerebellum or the pons where amyloid does not accumulate even with Alzheimer's disease, and the pixel values in the set reference region are used for normalizing data (refer to Non Patent Literature 1). For example, a mean standardized uptake value (SUV) of the reference region is obtained, and the reciprocal of the mean SUV is taken as a normalizing factor. The pixel value of each pixel in a nuclear medicine brain image is converted to an SUV, and the converted SUV is multiplied by the normalizing factor to obtain a map view.

### Citation List

### Non Patent Literature

Non Patent Literature 1: Yoshifumi Maya et al., "Preclinical and clinical properties of [123I]ABC577: A novel radioiodinated spect agent for imaging b-amyloid in the brain", Alzheimer's & Dementia: The Journal of the Alzheimer's Association, Volume 10, Issue 4, Supplement, Page P895, July 2014

### Summary

However, the inventors of the present application have discovered that, when a reference region is set on the cerebellum or the pons, a normalizing factor varies depending on the position or the size of the reference region. As a result, displayed images obtained by different analysts differ according to the difference in the setting of reference regions. This variation has made it difficult to compare images created by different analysts to evaluate the difference between the images. In order to study the progress of a disease or the effect of a treatment, a plurality of nuclear medical measurements need to be performed on the same patient at different times. However, different appearance of images for evaluation due to a difference in the setting of reference regions hinders correct evaluation on the progress of a disease or the effect of a treatment.

In view of the above situation, an object of the present invention is to provide a technique for evaluating a nuclear medicine brain image, the technique being hardly affected by a difference in the setting of reference regions.

The present invention relates to a technique for evaluating a nuclear medicine brain image. The most significant feature of the invention is to set a reference region on the scalp. An embodiment of the present invention generally includes:
- setting a reference region on a region corresponding to the scalp in the nuclear medicine brain image;
- calculating information on a pixel value in the set reference region; and
- normalizing, using the information, the pixel value of each pixel included in the nuclear medicine brain image or a value obtained from the pixel value, and outputting the normalized value.

The inventors of the present application have discovered that, when setting a reference region on the scalp to calculate information for normalization, the information is less dependent on the position or the size of the reference region. This configuration facilitates evaluation of the same nuclear medicine brain image by different image analysts, and also facilitates comparison and evaluation of a plurality of nuclear medicine brain images on the basis of a plurality of nuclear medical measurements performed on the same patient at different times.

The present invention is further advantageous when applied to amyloid imaging or receptor mapping in particular. The degrees of brain atrophy and diminished cerebral blood flow differ among elderly patients with dementia, who are primary targets of amyloid imaging or receptor imaging. The scalp, however, is not subject to the effect of brain atrophy or diminished cerebral blood flow. Thus, selecting the scalp as the basis of normalization enables evaluation of images that is less affected by the differences in brain atrophy and diminished cerebral blood flow among patients.

An example of preferred embodiments of the present invention is as follows:
A method for evaluating a nuclear medicine brain image, the method being executed by an apparatus when a program instruction stored in storage means of the apparatus is executed by processing means of the apparatus, the method comprising:
   setting a reference region on a region corresponding to the scalp in the nuclear medicine brain image, and storing information indicating the set reference region;
   based on the stored information indicating the reference region, calculating a value for normalization using a pixel included in a region in the nuclear medicine brain image corresponding to the reference region; and
   normalizing, using the calculated value for normalization, the pixel value of each pixel included in the nuclear medicine brain image or a value obtained from the pixel value, and outputting the normalized value.

Another example of preferred embodiments of the present invention is as follows:
An apparatus for evaluating a nuclear medicine brain image, the apparatus comprising:
   means for setting a reference region on a region corresponding to the scalp in the nuclear medicine brain image, and storing information indicating the set reference region;
   means for calculating, based on the stored information indicating the reference region, a value for normalization using a pixel included in a region in the nuclear medicine brain image corresponding to the reference region; and
   means for normalizing, using the calculated value for normalization, the pixel value of each pixel included in the nuclear medicine brain image or a value obtained from the pixel value, and outputting the normalized value.

Several preferred embodiments of the present invention are specified in the claims included in the attached claims. However, the embodiments specified in the claims do not necessarily include all the novel technical ideas disclosed in the present description and the drawings. The applicant claims to possess the right to have a patent granted on all the novel technical ideas disclosed in the present description and the drawings regardless of whether the novel technical ideas are claimed in the current claims.

### Brief Description of Drawings

FIG. 1 is a diagram illustrating a main configuration of an apparatus or a system 100, which is exemplary hardware capable of executing various processes disclosed in the present description.
FIG. 2 is a flowchart illustrating an outline of processes disclosed in the present description.
FIG. 3A is a flowchart illustrating an example of a reference region setting process.
FIG. 3B is a diagram illustrating a reference region that has been set using a morphologic image.
FIG. 4 is a flowchart illustrating an example of an evaluation information creating/outputting process.
FIG. 5 illustrates a reference region set in accordance with the present invention, and a corresponding SUVr image, with respect to an amyloid imaging image of a subject 1.
FIG. 6 illustrates another reference region set in accordance with the present invention, and a corresponding SUVr image, with respect to the same amyloid imaging image on the basis of which the images in FIG. 5 are created.
FIG. 7 illustrates a reference region set in accordance with the present invention, and a corresponding SUVr image, with respect to an amyloid imaging image of a subject 2.
FIG. 8 illustrates another reference region set in accordance with the present invention, and a corresponding SUVr image, with respect to the same amyloid imaging image on the basis of which the images in FIG. 7 are created.
FIG. 9 illustrates a reference region set in accordance with a conventional technique, and a corresponding SUVr image, with respect to the same amyloid imaging image on the basis of which the images in FIG. 5 and FIG. 6 are created.
FIG. 10 illustrates another reference region set in accordance with a conventional technique, and a corresponding SUVr image, with respect to the same amyloid imaging image on the basis of which the images in FIG. 5 and FIG. 6 are created.
FIG. 11 illustrates a reference region set in accordance with a conventional technique, and a corresponding SUVr image, with respect to the same amyloid imaging image on the basis of which the images in FIG. 7 and FIG. 8 are created.
FIG. 12 illustrates another reference region set in accordance with a conventional technique, and a corresponding SUVr image, with respect to the same amyloid imaging image on the basis of which the images in FIG. 7 and FIG. 8 are created.

### Description of Embodiments

The following describes examples of preferred embodiments of the present invention with reference to the accompanying drawings for deeper understanding of the present invention.

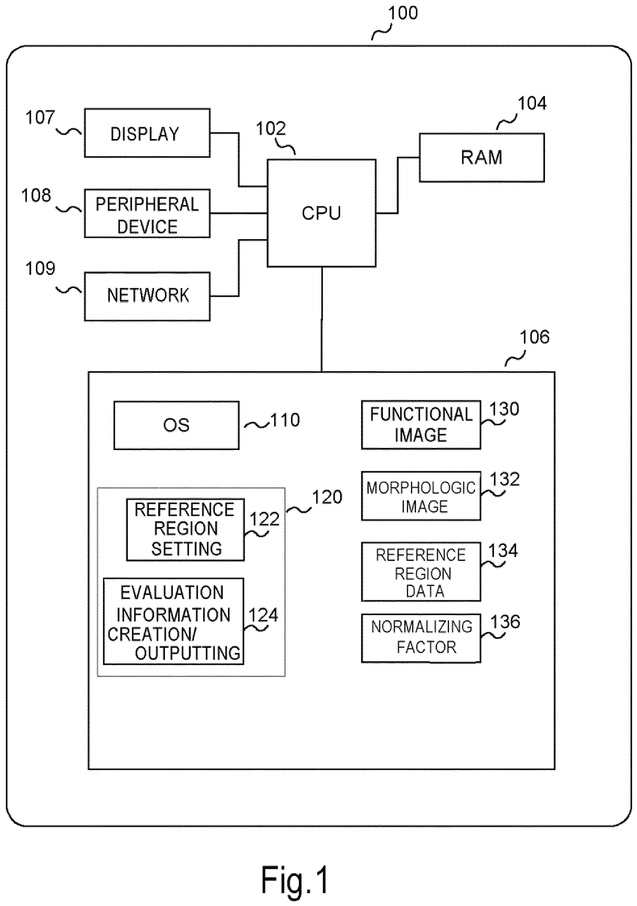

FIG. 1 is a diagram illustrating a main configuration of an apparatus or a system 100, which is exemplary hardware capable of executing various processes disclosed in the present description. As illustrated in FIG. 1, the system 100 is similar to a general-purpose computer in terms of hardware, and may include a central processing unit (CPU) 102, a main memory 104, an auxiliary storage unit 106, a display interface 107, a peripheral device interface 108, and a network interface 109, for example. Similarly to a general-purpose computer, a highspeed random access memory (RAM) may be used as the main memory 104, and an inexpensive, large-capacity hard disk or a solid state disk (SSD) may be used as the auxiliary storage unit 106. To the system 100, a display for displaying information may be connected through the display interface 107. Also to the system 100, a user interface such as a keyboard, a mouse, or a touch panel may be connected through the peripheral device interface 108. The network interface 109 may be used to connect the system 100 to another computer or the Internet via a network.

The auxiliary storage unit 106 may store therein, for example, an operating system (OS) 110 and a nuclear medicine brain image analysis program 120 for providing characteristic processes disclosed in the present description. The most basic functions of the system 100 are provided when the CPU 102 executes the OS 110.

In addition, novel processes disclosed in the present description are provided when the CPU 102 executes the nuclear medicine brain image analysis program 120. A program instruction group constructing the nuclear medicine brain image analysis program 120 may be programmed in any of existing computer program languages such as C++ and JAVA (registered trademark). The program instruction group may be converted to an executable format by a preferred compiler and stored in the auxiliary storage unit 106.

The auxiliary storage unit 106 may store therein, for example, a nuclear medicine brain image 130 subject to an analysis by the nuclear medicine brain image analysis program 120, a morphologic image 132 corresponding to the nuclear medicine brain image 130, and other pieces of data 134 and 136.

Other than the components illustrated in FIG. 1, the system 100 may include the same units as those of a general computer system, such as a power source and a cooling unit. Various embodiments of a computer system employing various techniques have been known, such as distributed, redundant, or virtualized storage units, use of multiple CPUs, CPU virtualization, use of a processor suitable for a specific process such as digital signal processing (DSP), and implementation of a specific process as hardware to be used with a CPU. The matters disclosed in the present description may be implemented in any form of computer system. The form of computer system does not limit the scope of the present invention. The matters disclosed in the present description are generally embodied as: (1) a computer program containing an instruction configured to cause, when executed by processing means, an apparatus or a system including the processing means to execute various processes described in the present description; (2) a method for operating the apparatus or the system, the method being performed when the processing means executes the computer program; and (3) the apparatus or the system including the computer program and the processing means configured to execute the computer program.

Note that the pieces of data 130 to 136 are not stored in the auxiliary storage unit 106 in many cases at the time of manufacture, sales, and initial start-up of the system 100. These pieces of data may be transferred from an external apparatus to the system 100 through the network interface 109, for example. In some embodiments, the pieces of data 134 and 136 may be generated and stored when the CPU 102 executes the computer program 120 or other computer programs. In some embodiments of the computer program 120 or the OS 110, the pieces of data 134 and 136 are stored only in the main memory 104 instead of being stored in the auxiliary storage unit 106. Note that the scope of the present invention is not limited by the existence of the pieces of data 130 to 136.

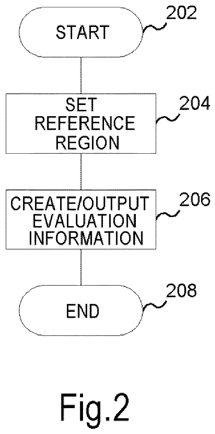

The following describes characteristic processes in a preferred example with reference to FIG. 2 and following diagrams. First, the nuclear medicine brain image 130 subject to an analysis in the present example is briefly described. The nuclear medicine brain image 130 may be, for example, image data created by amyloid imaging used for diagnosis of Alzheimer's disease. Amyloid imaging is a technique for imaging β-amyloid deposition, which is the main component of senile plaques seen in the brains of adult patients suspected to have Alzheimer's disease or other cognitive impairment. A radiopharmaceutical agent that binds to β-amyloid is intravenously injected into a patient's body and radiation emitted from the patient's body is detected with a positron emission tomography (PET) apparatus for imaging.

An image created by using a PET apparatus or a single-photon emission computed tomography (SPECT) apparatus is called a "functional image" due to its superior physiological information. (This is the reason for the image data 130 being described as "functional image" in the drawings of the present application.) By contrast, an image created by using a magnetic resonance imaging (MRI) apparatus or a computed tomography (CT) apparatus is called a "morphologic image" due to its superior anatomical information. The preferred embodiment of the present invention uses the morphologic image 132 in order to set a reference region in the nuclear medicine brain image 130 subject to an analysis. Apparatuses that integrate PET and MRI or PET and CT have recently been developed, thereby enabling imaging of a morphologic image along with the execution of amyloid imaging using PET. The pieces of image data 130 and 132 may be a functional image and a morphologic image, respectively, imaged together by such an apparatus.

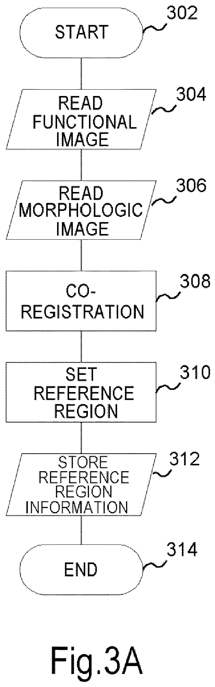

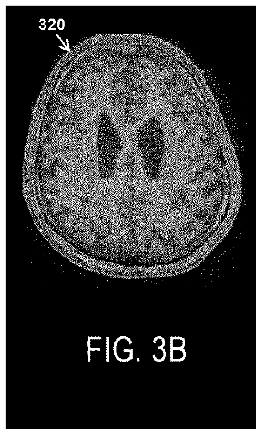

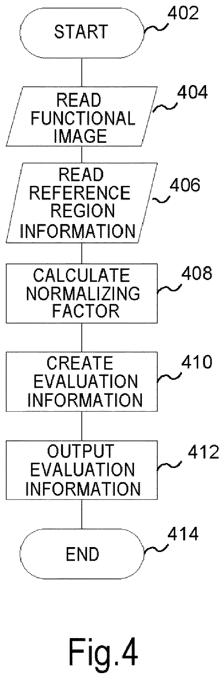

FIG. 2 is a flowchart illustrating an outline of processes according to a preferred embodiment of the present invention. The preferred embodiment is roughly divided into the following phases: a phase (204) of setting a reference region; and a phase (206) of creating and outputting information for evaluating the nuclear medicine brain image 130. These processes are described in detail with reference to FIG. 3 and FIG. 4, respectively. In some examples, the nuclear medicine brain image analysis program 120 may include dedicated modules (program instruction groups) to perform the processes at respective phases. For example, as in the present example, the nuclear medicine brain image analysis program 120 may include a reference region setting module (program instruction group) 122 to perform the process at the phase (204) of setting a reference region and an evaluation information creation/outputting module (program instruction group) 124 to perform the process at the phase (206) of creating and outputting evaluation information. In some examples, the nuclear medicine brain image analysis program 120 has no such a modular configuration.

FIG. 3A is a flowchart illustrating the details of the process at the phase (204) of setting a reference region illustrated in the flowchart of FIG. 2. In step 304, the nuclear medicine brain image 130 subject to an analysis in the present example is read. In other words, according to an instruction of the program instruction group contained in the nuclear medicine brain image analysis program 120, the CPU 102 copies at least part of the image data 130 from the auxiliary storage unit 106 to the main memory 104. In step 306, the data 132 that is a morphologic image is read.

In step 308, co-registration of the nuclear medicine brain image 130 and the morphologic image 132 is performed. In other words, the orientations, sizes, and positions of the nuclear medicine brain image 130 and the morphologic image 132 are adjusted to be the same. This process enables comparison between the nuclear medicine brain image 130 and the morphologic image 132. This process may simply be called registration. In the case where the pieces of image data 130 and 132 are imaged together by an apparatus that integrates PET or SPECT with MRI or CT, the co-registration of the nuclear medicine brain image 130 and the morphologic image 132 has often been completed by the time when the images are output from the apparatus. In this case, the process in step 308 is unnecessary. In some embodiments, the nuclear medicine brain image 130 and the morphologic image 132 may be modified to a standard head or a standard brain by the process in step 308 or another process not illustrated.

In step 310, a reference region is set by referring to the morphologic image 132 that has been co-registered. This reference region is used for the nuclear medicine brain image 130; however, recognition of anatomical information is easier with a morphologic image, and thus a morphologic image is used for setting a reference region in the preferred example. In some embodiments, a reference region can be set by modifying the nuclear medicine brain image 130 to a standard head or brain and applying a template reference region to the modified image. A reference region of the scalp may be set on the nuclear medicine brain image 130 by visual observation. In an aspect of setting a reference region directly on the nuclear medicine brain image 130, the above-described processes in steps 306 and 308 are unnecessary.

As described above, the present invention is characterized by the setting of a reference region on the scalp. The setting of a reference region may be performed manually or automatically. In an embodiment of manual setting, the reference region setting module (program instruction group) 122, when executed by the CPU 102, may cause the system 100 to execute a process including the following phases, for example:
(a) A phase of displaying a sectional image of the morphologic image 132. At this phase, the system 100 displays a desired sectional image of the morphologic image 132 on a display connected to the system 100 through the display interface 107. This display may include a touch panel.
(b) A phase of recording a trajectory of an input device such as a mouse, a stylus pen, or a finger of an operator. At this phase, when an operator traces a region to be set as a reference region (the entire scalp, for example) in the displayed sectional image with an input device, the system 100 records the trajectory.
(c) A phase of displaying the recorded trajectory on the sectional image in a superimposing manner.
(d) A phase of storing, as reference region information, the region surrounded by the recorded trajectory (in the main memory 104 or the auxiliary storage unit 106, for example). In an example, the reference region information may contain the same number of two-dimensional arrays as those of the sectional image displayed at (a). In the arrays, a first value may be assigned to the data (pixels) corresponding to the position of the trajectory and a second value to the data (pixels) not corresponding to the position of the trajectory. In another example, the reference region information may contain arrays in which a first value is assigned to the data of the region surrounded by the trajectory and a second value to the data of the region not surrounded by the trajectory. The reference region information may contain the position information on the sectional image displayed at (a), that is, the information for identifying the section on which the trajectory has been recorded. FIG. 1 exemplifies, as the reference region data 134, such reference region information that has been stored.

Examples of the sectional image and the trajectory displayed at (a) and (c), respectively, are illustrated in FIG. 3B. The displayed sectional image is an axial image imaged by MRI. As indicated by symbol 320, a site corresponding to the scalp is surrounded by a white line. This white line displays the trajectory traced by an operator with a mouse. In step 312, the system 100 stores, as the reference region data 134 for example, the information on the trajectory in the storage unit 106 according to the program instruction group contained in the reference region setting module. Step 314 indicates the end of the process.

FIG. 4 is a flowchart illustrating the details of the process at the phase (206) of creating and outputting information for evaluating the nuclear medicine brain image 130 illustrated in the flowchart of FIG. 2.

In step 404, the nuclear medicine brain image 130 subject to an analysis in the present example is read. In other words, according to an instruction of the program instruction group contained in the nuclear medicine brain image analysis program 120, the CPU 102 copies at least part of the image data 130 from the auxiliary storage unit 106 to the main memory 104. In step 406, the reference region data 134 that has been created and stored in steps 310 and 312, respectively, is read.

In step 408, a normalizing factor is calculated to be used for creating information for evaluating the nuclear medicine brain image 130. In some embodiments, for this calculation, the evaluation information creation/outputting module (program instruction group) 124, when executed by the CPU 102, may cause the system 100 to execute a process including the following phases, for example:
(a) Section information contained in the reference region data 134 is extracted. In other words, the section information on the morphologic image 132 used for creating the reference region data 134 is read.
(b) On the basis of the section information, a section is extracted from the nuclear medicine brain image 130. In other words, the same section as the section of the morphologic image 132 used for creating the reference region data 134 is cut out of the nuclear medicine brain image 130. The same section as the section of the morphologic image 132 used for creating the reference region data 134 can be cut out of the nuclear medicine brain image 130 on the basis of the section information contained in the reference region data 134 because the co-registration of the two images has been completed in step 308.
(c) On the basis of the reference region information on the reference region data 134, pixels and pixel values thereof are extracted to be used for calculating a normalizing factor. For example, when the reference region information contains the information on the trajectory traced in step 310, the pixels corresponding to positions surrounded by the trajectory are extracted from the section cut out at (b). For example, when the reference region information directly indicates positions inside the trajectory (and on the trajectory in some examples) traced in step 310, the pixels corresponding to the positions are extracted from the section cut out at (b).
(d) A normalizing factor is calculated from the pixels extracted at (c). In an example, the normalizing factor may be, for example, the reciprocal of the pixel mean value of the group of extracted pixels. In another example, the normalizing factor may be, for example, the reciprocal of the mean SUV of the group of extracted pixels.

In step 410, evaluation information used for evaluating the nuclear medicine brain image 130 is created using the normalizing factor calculated in step 408.

When the normalizing factor calculated in step 408 is the reciprocal of the pixel mean value of the reference region, the evaluation information may be obtained by multiplying the pixel value of each pixel in the nuclear medicine brain image 130 by the normalizing factor.

When the normalizing factor calculated in step 408 is the reciprocal of the mean SUV of the reference region, the evaluation information may be obtained by converting the pixel value of each pixel in the nuclear medicine brain image 130 to an SUV and multiplying the SUV by the normalizing factor. Such evaluation information may be described as SUVr, which means the ratio of SUV.

In step 412, the calculated evaluation information is output as a numeric value and/or an image. In the case of outputting an image, the calculated evaluation information alone may be output as an image. In some embodiments, however, the evaluation information may be displayed on the morphologic image in an overlapping manner. Step 414 indicates the end of the process.

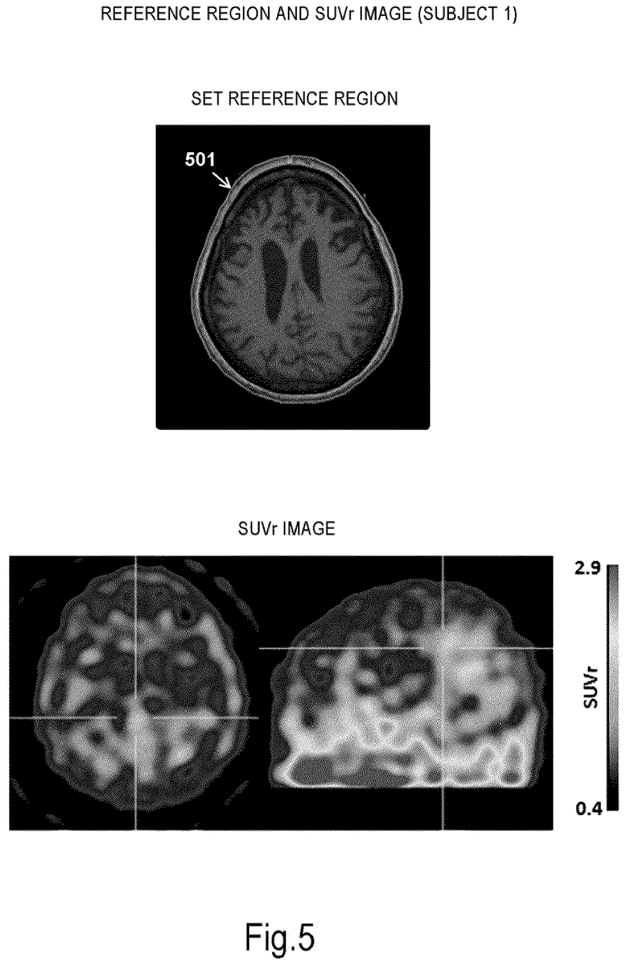

Examples of output from step 412 are illustrated in FIG. 5 to FIG. 8. Each diagram includes a set reference region and an SUVr image obtained on the basis of the reference region. As described above, each pixel value of the SUVr image is obtained by converting the pixel value of each pixel in the nuclear medicine brain image 130 to an SUV and multiplying the SUV by the normalizing factor.

FIG. 5 illustrates a set reference region 501 and an SUVr image obtained on the basis of the reference region 501, with respect to an amyloid imaging image of a subject (subject 1). The reference region 501 is a reference region that has been set by the same method as that described in relation to step 310 in FIG. 3A and FIG. 3B, in which an appropriate axial image is cut out of an MRI image and a site corresponding to the scalp is traced with a mouse or a stylus pen. The SUVr image in FIG. 5 is an image formed on the basis of the SUVr obtained in steps 404 to 410 on the basis of the reference region 501.

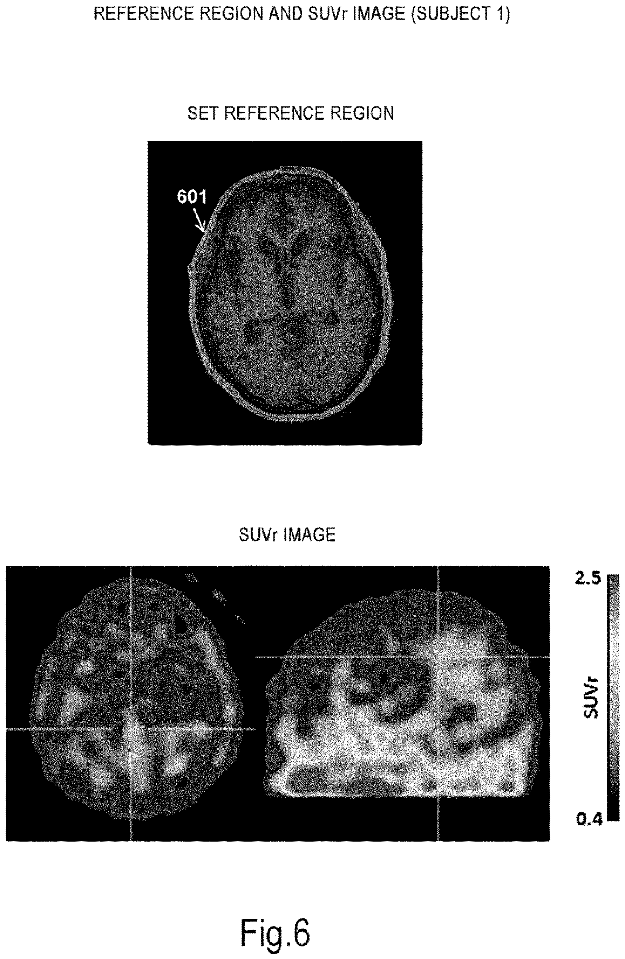

FIG. 6 illustrates an SUVr image created on the basis of the same functional image and morphologic image as those in FIG. 5. However, a set reference region is different from that in FIG. 5. As seen from the drawing, a reference region 601 set in the case of FIG. 6 has been created on the basis of an axial image different from the axial image on the basis of which the reference region 501 illustrated in FIG. 5 has been created. The reference region 601 has been set by tracing a site corresponding to the scalp in the axial image different from that in FIG. 5 with a mouse or a stylus pen.

Comparison between the SUVr image in FIG. 5 and the SUVr image in FIG. 6 reveals that similar shading can be observed. That is, it is observed that similar SUVr images are obtained regardless of the difference in the set reference regions.

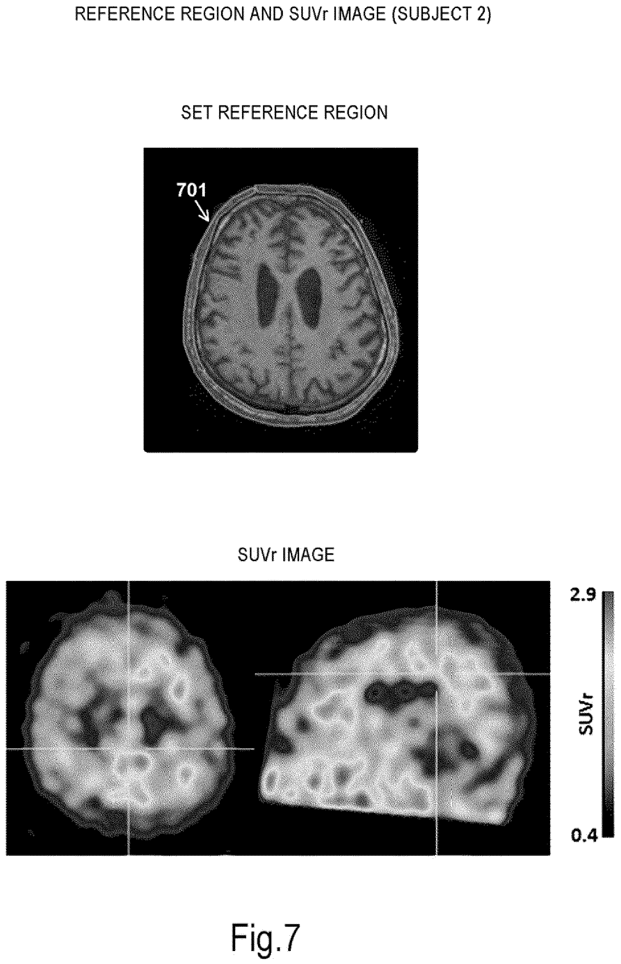

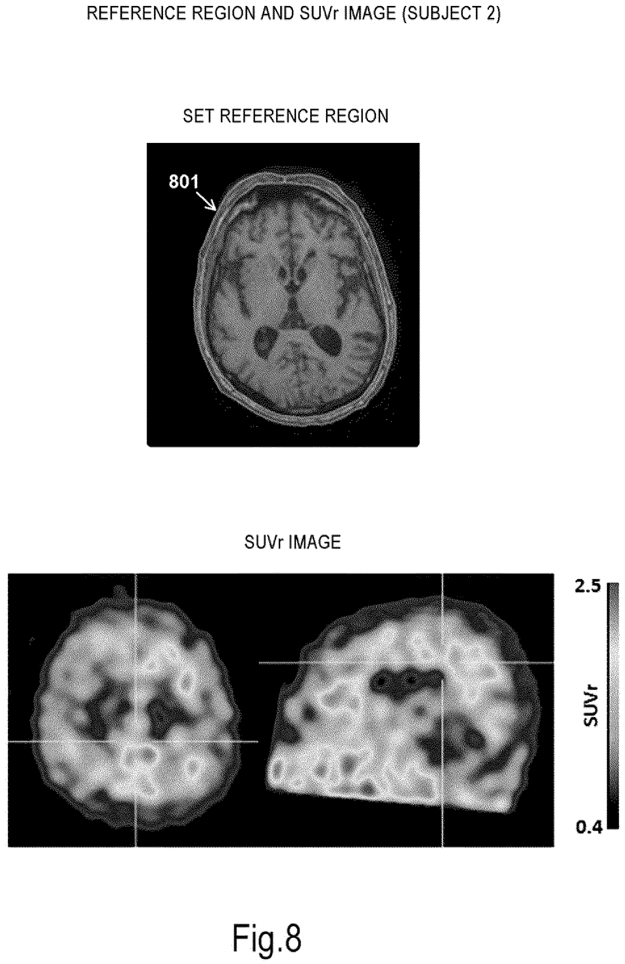

FIG. 7 and FIG. 8 illustrate the results of the same investigation on a different subject in order to indicate that similar effects can be obtained from different patients.

FIG. 7 illustrates a set reference region 701 and an SUVr image obtained on the basis of the reference region 701, with respect to an amyloid imaging image of a subject 2 different from the subject 1. The methods of setting the reference region and calculating the SUVr are the same as those in the case of FIG. 5 and FIG. 6. FIG. 8 illustrates a set reference region 801 and an SUVr image obtained on the basis of the reference region 801, with respect to the same amyloid imaging image as that in FIG. 7. As seen from the drawing, the reference region 801 has been created on the basis of an axial image different from the axial image on the basis of which the reference region 701 has been created.

Comparison between the SUVr image in FIG. 7 and the SUVr image in FIG. 8 reveals that similar shading can be observed. That is, it is confirmed also on the subject 2 that similar SUVr images are obtained regardless of the difference in the set reference regions.

As a reminder, the following describes some of the conditions of the images on the basis of which the images in FIG. 5 to FIG. 8 have been created. As described above, the images in FIG. 5 and FIG. 6 have been created from the same amyloid imaging image. This amyloid imaging image was obtained by administering the radiopharmaceutical agent ABC577 to the subject 1 at a radioactivity dose of 195 MBq. SPECT measurement and MRI measurement were then performed sequentially. The obtained SPECT image and MRI image were aligned with each other and were used for the subsequent processes. The section used for the reference region is positioned at the 52nd slice from the vertex in the case of FIG. 5. The slice has a thickness of 1.067 mm, which corresponds to a thickness of approximately 5.55 cm when converted to the measurement of the actual head. In the case of FIG. 6, the section used for the reference region is positioned at the 83rd slice from the vertex.

As described above, the images in FIG. 7 and FIG. 8 have also been created from the same amyloid imaging image. This amyloid imaging image was obtained by administering the radiopharmaceutical agent ABC577 to the subject 2 at a radioactivity dose of 198 MBq. SPECT measurement and MRI measurement were then performed sequentially. The obtained SPECT image and MRI image were aligned with each other and were used for the subsequent processes. The section used for the reference region is positioned at the 52nd slice from the vertex in the case of FIG. 7, and at the 80th slice from the vertex in the case of FIG. 8.

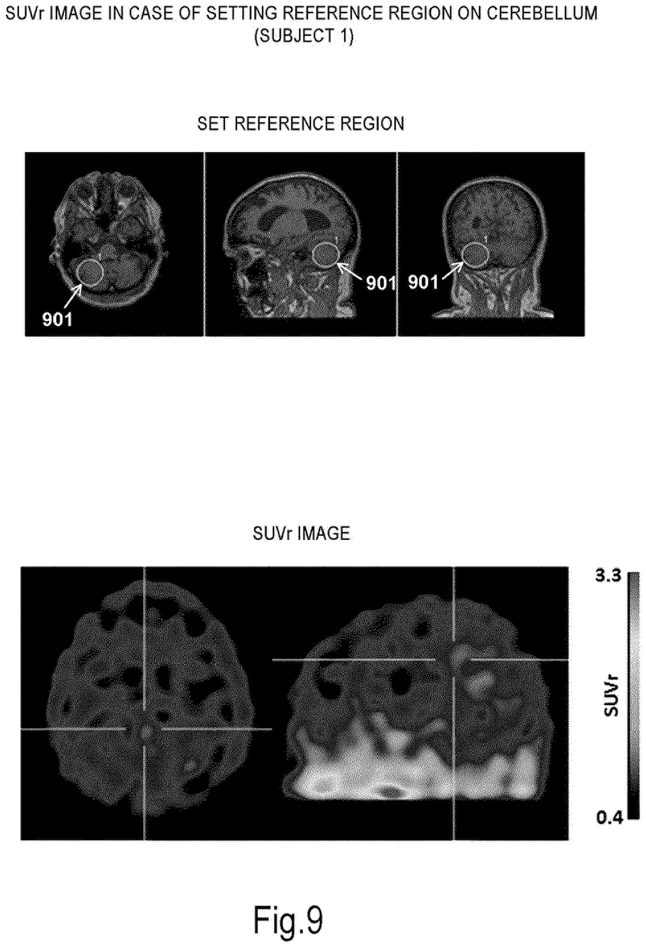

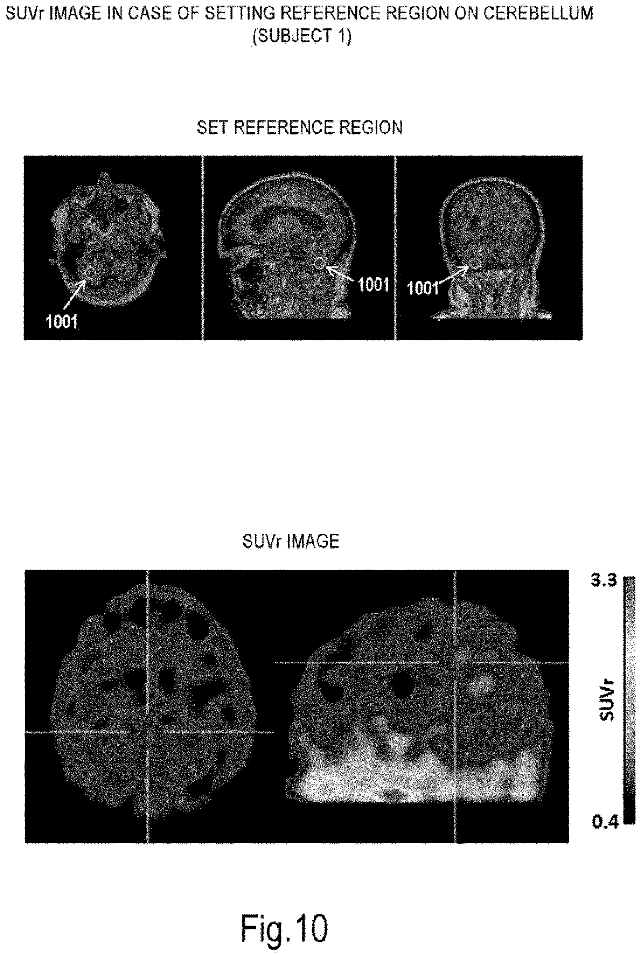

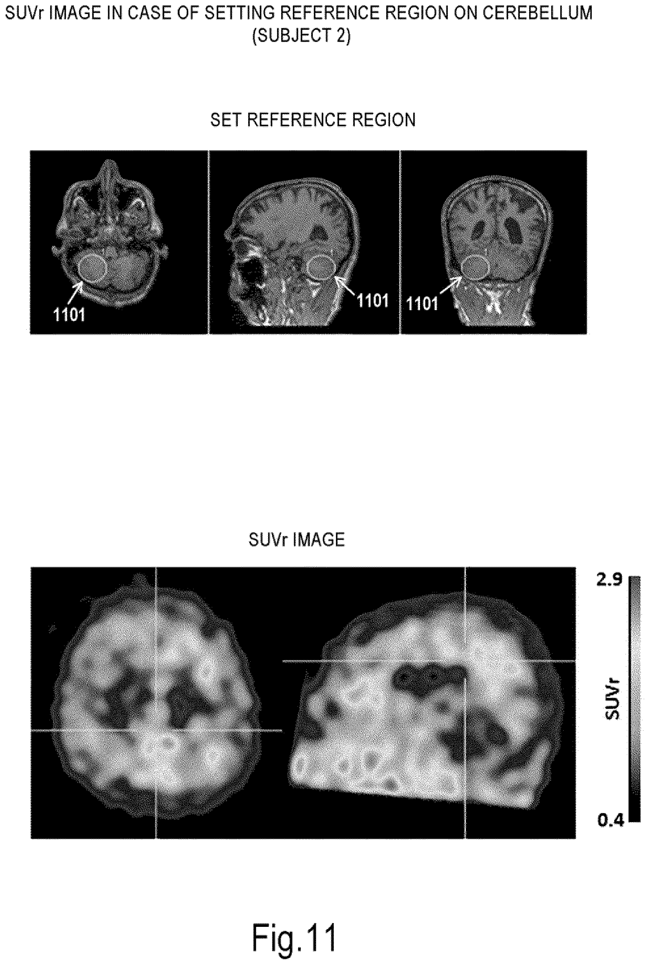

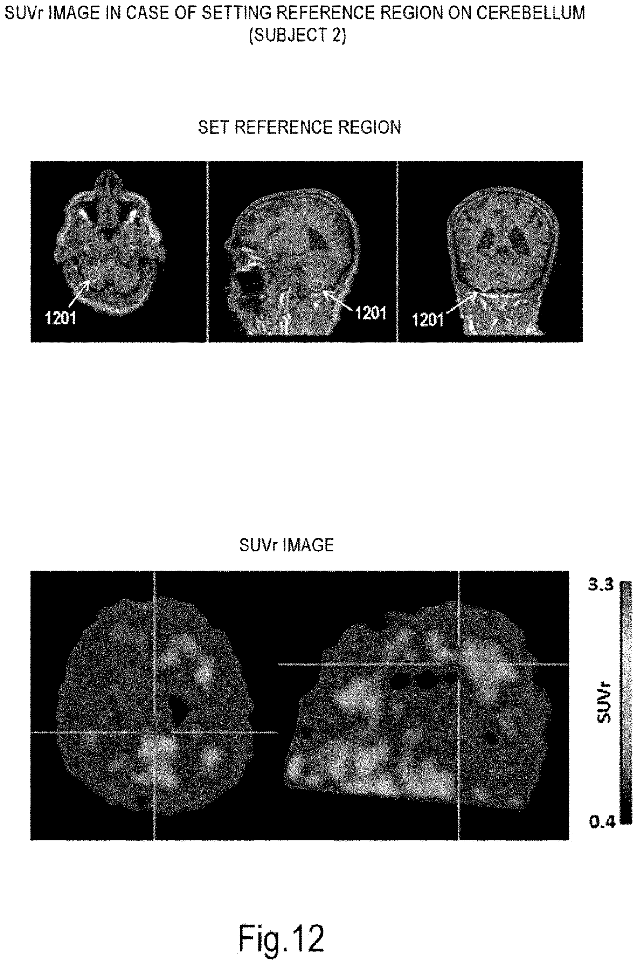

FIG. 9 to FIG. 12 illustrate SUVr images obtained when reference regions are set on the cerebellum as in conventional techniques in order to compare the effects of the present invention with those of a conventional technique. FIG. 9 illustrates a region denoted by symbol 901 that has been set as a reference region, and an image obtained by calculating an SUVr, with respect to the same amyloid imaging image as that in the cases of FIG. 5 and FIG. 6. FIG. 10 illustrates a region denoted by symbol 1001 that has been set as a reference region, and an image obtained by calculating an SUVr, with respect to the same amyloid imaging image as that in the cases of FIG. 5 and FIG. 6 (and FIG. 9). FIG. 11 illustrates a region denoted by symbol 1101 that has been set as a reference region, and an image obtained by calculating an SUVr, with respect to the same amyloid imaging image as that in the cases of FIG. 7 and FIG. 8. FIG. 12 illustrates a region denoted by symbol 1201 that has been set as a reference region, and an image obtained by calculating an SUVr, with respect to the same amyloid imaging image as that in the cases of FIG. 7 and FIG. 8 (and FIG. 11).

Comparison between the reference region 901 in FIG. 9 and the reference region 1001 in FIG. 10 reveals that although both regions are set on the cerebellum, the sizes of the regions are significantly different from each other. Similarly, comparison between the reference region 1101 in FIG. 11 and the reference region 1201 in FIG. 12 reveals that although both regions are set at the cerebellum, the sizes of the regions are significantly different from each other.

Comparison between the SUVr image in FIG. 9 and the SUVr image in FIG. 10 reveals that similar shading can be observed on both images. Thus, there are cases in which similar SUVr images can be obtained from differently set reference regions in accordance with a conventional technique.

However, comparison between the SUVr image in FIG. 11 and the SUVr image in FIG. 12 reveals that the images have significantly different shading.

As described above, a conventional technique may result in an SUVr image that is significantly different depending on the way of setting its reference region. Such a phenomenon has first been discovered by the inventors of the present application. The present invention has been made to solve such a problem.

Setting a reference region on the scalp according to the present invention and normalizing a nuclear medicine brain image can stably provides similar evaluation images regardless of the positions or the sizes of reference regions. This configuration facilitates evaluation of the same nuclear medicine brain image by different image analysts, and also facilitates comparison and evaluation of a plurality of nuclear medicine brain images on the basis of a plurality of nuclear medical measurements performed on the same patient at different times.

As described above, the present invention is further advantageous when applied to amyloid imaging in particular. The degrees of brain atrophy and diminished cerebral blood flow differ among elderly patients with dementia, who are targets of amyloid imaging. The scalp, however, is not subject to the effect of brain atrophy or diminished cerebral blood flow. Thus, selecting the scalp as the basis of normalization enables evaluation of images that is less affected by the differences in brain atrophy and diminished cerebral blood flow among patients.

However, it is emphasized that the field of application of the present invention is not limited to amyloid imaging, and the present invention is widely applicable to cases where normalization of nuclear medicine images is necessary. As described above, the scalp is not subject to the effect of brain atrophy or diminished cerebral blood flow. Thus, selecting the scalp as the basis of normalization is an exceptional idea. The present invention enables evaluation of images that is less affected by the differences in brain atrophy and diminished cerebral blood flow among patients, in various applications.

Preferred embodiments of the present invention have been described above; however, it is needless to say that the embodiments of the present invention are not limited to those described above. The above-described embodiments are merely examples. The present invention may have various other embodiments.

For example, although a manual method has been described as a method for setting a reference region on the scalp, an embodiment that sets a reference region automatically is also applicable. In an embodiment of automatic setting, the reference region setting module (program instruction group) 122, when executed by the CPU 102, may cause the system 100 to execute a process including the following phases, for example:
(a) A phase of cutting a specific section out of the morphologic image 132. This section is preferably an axial section on which the scalp is easily identified.
(b) Changes in pixel values are scanned radially from the center of the image, and the farthest point in each scanning direction at which the pixel value is lower than a threshold is set as the outer boundary point of the scalp.
(c) Inside the outer boundary point of the scalp in each scanning direction described above, the first point at which the pixel value becomes lower than the threshold is set as the inner boundary point of the scalp.
(d) The outer boundary points are connected to one another and so are the inner boundary points to define the boundaries of the scalp, and the inside between the boundaries (inclusive of the boundaries, in some embodiments) is set as a reference region.

Performing the process including the above-described phases (a) to (d) on a plurality of sections also enables setting of a three-dimensional reference region.

Other than the embodiments described above, the present invention may have various embodiments.

Individual features included in the various examples that have been described in the description or the drawings are not limited to usage with examples in which these features are explicitly explained to be included, but may be used in combination with other examples that have been described herein or various specific examples that have not been described. In particular, the processes presented in the flowcharts do not necessarily need to be performed in the described order. According to the preference of an executor, the processes may be performed in a changed order or in parallel, or as a plurality of blocks integrally implemented, or in a loop as appropriate. These variations are all included in the scope of the present invention. The order of the description of the processes defined in the claims does not necessarily specify the mandatory order of the processes. For example, an embodiment specifying a different order of the processes and an embodiment that executes the processes in a loop are also included in the scope of the present invention according to the claims. It should be noted that the applicant claims to possess the right to have the patent granted on all the embodiments not deviating from the spirit of the present invention regardless of whether a patent is claimed in the current set of attached claims.

### Reference Signs List

- 100: system
- 104: main memory
- 106: auxiliary storage unit
- 107: display interface
- 108: peripheral device interface
- 109: network interface
- 110: OS
- 120: nuclear medicine brain image analysis program
- 130: nuclear medicine brain image data

## Claims

1. A method for evaluating a nuclear medicine brain image, the method being executed by an apparatus when a program instruction stored in storage means of the apparatus is executed by processing means of the apparatus, the method comprising:
setting a reference region on a region corresponding to a scalp in the nuclear medicine brain image, and storing information indicating the set reference region;
based on the stored information indicating the reference region, calculating a value for normalization using a pixel included in a region in the nuclear medicine brain image corresponding to the reference region; and
normalizing, using the calculated value for normalization, a pixel value of each pixel included in the nuclear medicine brain image or a value obtained from the pixel value, and outputting the normalized value.

2. The method according to claim 1, wherein the outputting includes displaying the normalized value in a map view.

3. The method according to claim 2, further comprising setting the reference region using a morphologic image different from the nuclear medicine brain image.

4. The method according to claim 3, wherein
the information indicating the reference region includes information on a section of the morphologic image used to set the reference region,
the method further comprising extracting a pixel for calculating the value for normalization from the nuclear medicine brain image by using the information on the section.

5. A computer program comprising a program instruction that causes, when executed by processing means of an apparatus, the apparatus to execute the method according to any one of claims 1 to 4.

6. An apparatus comprising:
processing means; and
means for storing therein a program instruction,
the program instruction causing, when executed by the processing means, the apparatus to execute the method according to any one of claims 1 to 4.

7. An apparatus for evaluating a nuclear medicine brain image, the apparatus comprising:
means for setting a reference region on a region corresponding to a scalp in the nuclear medicine brain image, and storing information indicating the set reference region;
means for calculating, based on the stored information indicating the reference region, a value for normalization using a pixel included in a region in the nuclear medicine brain image corresponding to the reference region; and
means for normalizing, using the calculated value for normalization, a pixel value of each pixel included in the nuclear medicine brain image or a value obtained from the pixel value, and outputting the normalized value.
